(19) Europäisches Patentamt
European Patent Office
Office européen des brevets

(11) EP 4 209 542 B1

(12) EUROPEAN PATENT SPECIFICATION

(45) Date of publication and mention of the grant of the patent:
**05.08.2026 Bulletin 2026/32**

(21) Application number: **22828757.9**

(22) Date of filing: **22.06.2022**

(51) International Patent Classification (IPC):
***C08K 5/103*** *(2006.01)* ***C08K 5/00*** *(2006.01)*
***C07C 67/08*** *(2006.01)* ***C07C 69/30*** *(2006.01)*
***C07C 69/74*** *(2006.01)* ***C11C 3/02*** *(2006.01)*

(52) Cooperative Patent Classification (CPC):
(C-Sets available)
**C08K 5/103; C07C 69/30; C07C 69/74;**
C07C 2601/08; C08K 5/0016 (Cont.)

(86) International application number:
**PCT/KR2022/008885**

(87) International publication number:
**WO 2022/270910 (29.12.2022 Gazette 2022/52)**

# (54) TRIESTER-BASED PLASTICIZER COMPOSITION AND RESIN COMPOSITION COMPRISING THE SAME

TRIESTERBASIERTE WEICHMACHERZUSAMMENSETZUNG UND HARZZUSAMMENSETZUNG DAMIT

COMPOSITION DE PLASTIFIANT À BASE D'ISOPHTALATE ET COMPOSITION DE RÉSINE LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2021 KR 20210080755**

(43) Date of publication of application:
**12.07.2023 Bulletin 2023/28**

(73) Proprietor: **LG Chem, Ltd.**
**Seoul 07336 (KR)**

(72) Inventors:
- **KIM, Hyun Kyu**
  **Daejeon 34122 (KR)**
- **KIM, Joo Ho**
  **Daejeon 34122 (KR)**
- **CHOI, Woo Hyuk**
  **Daejeon 34122 (KR)**
- **MOON, Jeong Ju**
  **Daejeon 34122 (KR)**
- **JEONG, Seok Ho**
  **Daejeon 34122 (KR)**

(74) Representative: **Plasseraud IP**
**104 Rue de Richelieu**
**CS92104**
**75080 Paris Cedex 02 (FR)**

(56) References cited:
**WO-A1-03/054106**
**WO-A1-2010/027640**
**WO-A1-2022/270911**
**WO-A2-2011/139016**
**JP-A- H11 349 766**
**KR-A- 20040 071 179**
**KR-A- 20110 122 571**
**US-A1- 2010 249 299**
**US-A1- 2023 374 262**
**US-A2- 2012 022 197**
**US-B2- 8 299 281**
**US-B2- 8 771 815**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08K 5/103, C08L 27/06**

## Description

## TECHNICAL FIELD

### Cross-reference to Related Applications

The present application claims the benefit of priority based on Korean Patent Application No. 10-2021-0080755, filed on June 22, 2021,

### Technical Field

The present invention relates to a plasticizer composition including one or more triester groups, and a resin composition comprising the same.

## BACKGROUND ART

Generally, plasticizers are obtained through the reaction of alcohols with polycarboxylic acids such as phthalic acid and adipic acid for forming corresponding esters. In addition, considering the internal and external regulations on harmful phthalate-based plasticizers to the human body, studies are being continued on plasticizer compositions which may replace phthalate-based plasticizers such as terephthalate-based, adipate-based and other polymer-based plasticizers.

Meanwhile, regardless of the type of industry including plastisol type of industry of flooring materials, wallpaper, soft and hard sheets, etc., calendaring type of industry, or extrusion/injection compound type of industry, the demand for eco-friendly products is increasing. In order to reinforce the quality properties, processability and productivity by the finished products, a suitable plasticizer is required considering discoloration, migration, mechanical properties, etc.

According to the properties required by the types of industry in various areas of usage, such as tensile strength, elongation rate, light resistance, migration, gelling properties and absorption rate, supplementary materials such as a plasticizer, a filler, a stabilizer, a viscosity decreasing agent, a dispersant, a defoaming agent and a foaming agent are mixed with a PVC resin.

For example, in case of applying di(2-ethylhexyl) terephthalate (DEHTP) which is relatively cheap and widely used among plasticizer compositions which may be applied to PVC, hardness or sol viscosity is high, absorption rate of a plasticizer is relatively slow, and migration and stress migration are not good.

As improvements on the above limitations, the application of a transesterification product with butanol as a plasticizer, as a composition including DEHTP may be considered. However, plasticization efficiency is improved but volatile loss or thermal stability is inferior and mechanical properties are somewhat degraded, and the improvement of physical properties is required. Accordingly, generally, there is no solution but employing a method compensating the defects through mixing with a different second plasticizer at the present time.

However, in case of applying the second plasticizer, there are drawbacks of generating unexpected defects as follows: the change of the physical properties is hard to predict; the application may become a factor of increasing the unit cost of the product; the improvement of the physical properties is not clearly shown except for specific cases; and problems relating to compatibility with a resin may arise.

In addition, if a material like tri(2-ethylhexyl) trimellitate or triisononyl trimellitate is applied as a trimellitate-based product in order to improve the inferior migration and loss properties of the DEHTP products, migration or loss properties may be improved, but plasticization efficiency may be degraded, and a great deal of the material is required to inject to provide a resin with suitable plasticization effects, and considering the relatively high unit price of the products, commercialization thereof is impossible.

Accordingly, the development of products for solving the environmental issues of the conventional phthalate-based products or products for improving inferior physical properties of the eco-friendly products for improving the environmental issues of the phthalate-based products is required.

## DISCLOSURE OF THE INVENTION

## TECHNICAL PROBLEM

The present invention is to provide a plasticizer composition which may maintain the equal levels of migration resistance and volatile loss as those applying the conventional plasticizer, while markedly improving mechanical properties, absorption rate, stress migration and plasticization efficiency, by including triesters which are products derived from the esterification of a hexanoic acid isomer mixture and a trihydric alcohol.

## TECHNICAL SOLUTION

To solve the tasks, the present invention provides a plasticizer composition and a resin composition.

(1) The present invention provides a triester-based plasticizer composition, comprising one or more triesters of the following Formula 1, wherein $R_1$ to $R_3$ of Formula 1 are derived from a hexanoic acid isomer mixture having a degree of branching of 2.0 or less:

[Formula 1]

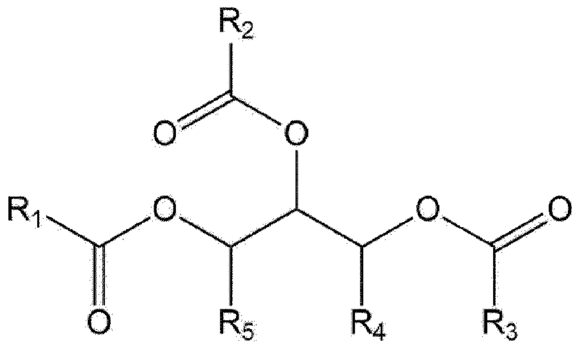

in Formula 1,

$R_1$ to $R_3$ are each independently an n-pentyl group, a branch-type pentyl group or a cyclopentyl group, and $R_4$ and $R_5$ are each independently hydrogen or an alkyl group of 1 to 4 carbon atoms.

(2) The present invention provides the plasticizer composition according to (1), wherein the hexanoic acid isomer mixture has the degree of branching of 1.5 or less.
(3) The present invention provides the plasticizer composition according to (1) or (2), wherein the hexanoic acid isomer mixture comprises 2-methylpentanoic acid and 3-methylpentanoic acid.
(4) The present invention provides the plasticizer composition according to any one of (1) to (3), wherein the hexanoic acid isomer mixture comprises 1-hexanoic acid, 2-methylpentanoic acid, 3-methylpentanoic acid and cyclopentyl methanoic acid.
(5) The present invention provides the plasticizer composition according to any one of (1) to (4), wherein the hexanoic acid isomer mixture comprises 20 to 95 parts by weight of a branch-type hexanoic acid with respect to 100 parts by weight of the mixture.
(6) The present invention provides the plasticizer composition according to any one of (1) to (5), wherein the hexanoic acid isomer mixture comprises 30 parts by weight or less of cyclopentyl methanoic acid with respect to 100 parts by weight of the mixture.
(7) The present invention provides the plasticizer composition according to any one of (1) to (6), wherein the hexanoic acid isomer mixture comprises 80 parts by weight or less of 1-hexanic acid with respect to 100 parts by weight of the mixture.
(8) The present invention provides the plasticizer composition according to any one of (1) to (7), wherein $R_4$ and $R_5$ are hydrogen.
(9) The present invention provides a resin composition comprising: 100 parts by weight of a resin; and 5 to 150 parts by weight of the plasticizer composition according to any one of (1) to (8).
(10) The present invention provides the resin composition according to (9), wherein the resin is one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, natural rubber, and synthetic rubber.

## ADVANTAGEOUS EFFECTS

The plasticizer composition according to an embodiment of the present invention, if used in a resin composition, may maintain the equal levels of migration resistance and volatile loss in contrast to the conventional plasticizer and may markedly improve mechanical properties, absorption rate, stress migration and plasticization efficiency.

## MODE FOR CARRYING OUT THE INVENTION

It will be understood that terms or words used in the present disclosure and claims should not be interpreted as having a meaning that is defined in common or in dictionaries, however should be interpreted in consistent with the technical scope of the present invention based on the principle that inventors may appropriately define the concept of the terms to explain the invention at his best method.

### Definition of terms

The term "composition" as used in the present disclosure includes a mixture of materials including the corresponding composition as well as a reaction product and a decomposition product formed from the materials of the corresponding composition.

The term "isomer" as used in the present disclosure does not intend to differentiate all meanings of isomers but intends to mean structural isomers, that is, the relation having the same carbon number but different bonding structures so as to differentiate these types, and does not mean materials differentiated as stereoisomers such as enantiomers and diastereomers.

The term "straight vinyl chloride polymer" as used in the present disclosure may be one type of vinyl chloride polymers and polymerized by suspension polymerization, bulk polymerization, etc., and may refer to a polymer having a porous particle shape in which a large number of pores having a size of tens to hundreds of micrometers, no cohesiveness, and excellent flowability are dispersed.

The term "paste vinyl chloride polymer" as used in the present disclosure may be one type of vinyl chloride polymers and polymerized by microsuspension polymerization, microseed polymerization, emulsion polymerization, etc., and may refer to a polymer having minute particles without pores and a size of tens to thousands of nanometers, cohesiveness, and inferior flowability.

The terms "comprising", and "having" and the derivatives thereof in the present invention, though these terms are particularly disclosed or not, do not intended to preclude the presence of optional additional components, steps, or processes. In order to avoid any uncertainty, all compositions claimed by using the term "comprising" may include optional additional additives, auxiliaries, or compounds, including a polymer or any other materials, unless otherwise described to the contrary. In contrast, the term "consisting essentially of ~" excludes unnecessary ones for operation and precludes optional other components, steps or processes from the scope of optional continuous description. The term "consisting of ~" precludes optional components, steps or processes, which are not particularly described or illustrated.

### Measurement methods

In the present disclosure, the content analysis of the components in a composition is conducted by gas chromatography measurement using a gas chromatography equipment of Agilent Co. (product name: Agilent 7890 GC, column: HP-5, carrier gas: helium (flow rate of 2.4 ml/min), detector: F.I.D., injection volume: 1 μl, initial value: 70°C/4.2 min, end value: 280°C/7.8 min, program rate: 15°C/min).

In the present disclosure, "hardness" means Shore hardness (Shore "A" and/or Shore "D") at 25°C and is measured in conditions of 3T 10s using ASTM D2240. The hardness may be an index for evaluating plasticization efficiency, and the lower the value is, the better the plasticization efficiency is.

In the present disclosure, "tensile strength" is obtained according to an ASTM D638 method by drawing a specimen in a cross head speed of 200 mm/min (1T) using a test apparatus of U.T.M (manufacturer: Instron, model name: 4466), measuring a point where the specimen is cut, and calculating according to the following Mathematical Formula 1:

Tensile strength ($kgf/cm^2$) = load value (kgf)/thickness (cm) $\times$ width (cm) [Mathematical Formula 1]

In the present disclosure, "elongation rate" is obtained according to an ASTM D638 method by drawing a specimen in a cross head speed of 200 mm/min (1T) using the U.T.M, measuring a point where the specimen is cut, and calculating according to the following Mathematical Formula 2:

Elongation rate (%) = length after elongation/initial length $\times$ 100 [Mathematical Formula 2]

In the present disclosure, "migration loss" is obtained according to KSM-3156, by which a specimen with a thickness of 2 mm or more is obtained, glass plates are attached onto both sides of the specimen and a load of 1 $kgf/cm^2$ is applied. The specimen is stood in a hot air circulation type oven (80°C) for 72 hours, then taken out therefrom and cooled at room temperature for 4 hours. Then, the glass plates attached onto both sides of the specimen are removed, the weights

before and after standing the glass plates and the specimen plate in the oven are measured, and the migration loss is calculated according to Mathematical Formula 3 below.

Migration loss (%) = {[(weight of initial specimen) - (weight of specimen after standing in oven)]/(weight of initial specimen) } × 100 [Mathematical Formula 3]

In the present disclosure, "volatile loss" is obtained by processing a specimen at 80°C for 72 hours and then, measuring the weight of the specimen.

Volatile loss (wt%) = {[(weight of initial specimen) - (weight of specimen after processing)]/(weight of initial specimen) } × 100 [Mathematical Formula 4]

In case of the various measurement conditions, the details of the conditions of the temperature, the speed of revolution, the time, etc., may be somewhat changed according to situations, and if the conditions are different, a measurement method and its conditions are required to be separately indicated.

Hereinafter, the present invention will be explained in more detail to assist the understanding of the present invention.

According to an embodiment of the present invention, a plasticizer composition includes one or more triesters of Formula 1 below, wherein the alkyl groups of the triester are derived from a hexanoic acid isomer mixture having a degree of branching of 2.0 or less.

[Formula 1]

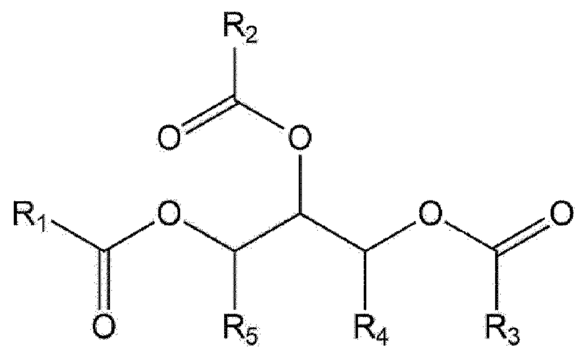

In Formula 1, $R_1$ to $R_3$ are each independently an n-pentyl group, a branch type pentyl group or a cyclopentyl group, and $R_4$ and $R_5$ are each independently hydrogen or an alkyl group of 1 to 4 carbon atoms.

The plasticizer composition may be a product produced by the esterification of a hexanoic acid isomer mixture and a trihydric alcohol, and accordingly, may be derived from a carboxylic acid having a carbon chain with 6 carbon atoms including a carbonyl group as central carbon. As $R_1$ to $R_3$ of Formula 1, a linear, branch-type or alicyclic alkyl group with 5 carbon atoms may be applied.

The plasticizer composition according to an embodiment of the present invention includes one or more triesters represented by Formula 1, wherein the number of triesters finally produced may be determined according to the number of hexanoic acids included in the hexanoic acid isomer mixture applied for the esterification. For example, if two types of isomers are included in the hexanoic acid isomer mixture, at least five types of triesters may be included in the plasticizer composition, and if three types of isomers are included, at least 15 types of triesters may be included in the plasticizer composition.

In the plasticizer composition according to an embodiment of the present invention, specifically alkyl carboxylic acid with 6 carbon number, i.e., hexanoic acid is applied, and plasticization efficiency and mechanical properties may be improved simultaneously in contrast to a case applying other carbon number. If alkyl carboxylic acid with 5 or less carbon number is applied, mechanical properties and stress migration might be degraded, and if alkyl carboxylic acid with 7 or more carbon number is applied, plasticization efficiency may be inferior, absorption rate may be very slow, and processability might be markedly deteriorated.

In addition, considering a compound with three ester groups as the triester, the plasticizer composition has excellent compatibility with a resin and excellent miscibility with other additives, and has lots of ester groups to immobilize a molecule in a polymer chain, and accordingly, may have excellent plasticization efficiency and mechanical properties, while maintaining suitable levels of migration resistance and volatile loss.

Further, different from a case where a benzene ring is present in a molecule as in a petroleum-based plasticizer, there is no benzene ring in a molecule, and the plasticizer may be classified into an eco-friendly plasticizer and evaluated to have excellent performance when compared to the petroleum-based plasticizer. These effects are understood due to the preparation of the plasticizer from the reaction of a polyhydric alcohol and a monocarboxylic acid, different from the conventional preparation of a plasticizer from the reaction of a polyfunctional acid and a monoalcohol.

The alkyl group of the triester included in the plasticizer composition according to an embodiment of the present invention may be derived from a hexanoic acid isomer mixture having a degree of branching of 2.0 or less, preferably, the degree of branching of 1.5 or less, 1.3 or less, 1.2 or less, or 1.0 or less. In addition, the degree of branching may be 0.1 or more, 0.2 or more, 0.3 or more.

Here, the degree of branching may mean that how many branch carbon atoms do the alkyl groups bonded to a material included in the composition have, and may be determined according to the weight ratio of the corresponding material. For example, if 60 wt% of 1-hexanoic acid, 30 wt% of 2-methylpentanoic acid, and 10 wt% of 2-ethyl butanoic acid are included in a hexanoic acid mixture, the branch carbon numbers of each carboxylic acid are 0, 1 and 2, respectively, and the degree of branching may be calculated by [(60x0)+(30x1)+(10x2)]/100, and may be 0.5. Meanwhile, in the present invention, the branch carbon number of cyclopentyl methanoic acid is regarded 0.

Specifically, according to the features that what ratio of a branch-type alkyl group is present among total alkyl radicals, further, what ratio of a specific branch-type alkyl radicals are present among the branch-type alkyl groups, plasticization efficiency and the physical properties of migration resistance/volatile loss may be balanced even further, and processability may be optimized. In addition, according to the interaction among multiple triesters included in the composition, marked improvement of mechanical properties such as tensile strength and elongation rate, and stress resistance may be achieved.

Through this, a material completely free from environmental issues, and at the same time, a product markedly improving the tensile strength of the conventional phthalate-based products could be accomplished, migration resistance and stress resistance of the conventional terephthalate-based products may be markedly improved, and a product having balanced physical properties and markedly improved levels thereof in contrast to the conventional commercial products could be achieved.

According to an embodiment of the present invention, for optimal and favorable accomplishment of the above-described effects, the hexanoic acid isomer mixture may include essentially 2-methylpentanoic acid and 3-methylpentanoic acid. By including essentially the two isomers among various isomers in the isomer mixture, the above-described effects could be achieved with even higher reproducibility.

In addition, the hexanoic acid isomer mixture may further include 1-hexanoic acid and cyclopentyl methanoic acid in addition to the 2-methylpentanoic acid and 3-methylpentanoic acid. In the case of 1-hexanoic acid, specific physical properties tend to improve with the inclusion thereof, but the amount is required to be controlled considering absorption rate or the processability of plasticization efficiency, and the same may be applied for cyclopentyl methanoic acid.

In the plasticizer composition according to an embodiment of the present invention, in the hexanoic acid isomer mixture, branch-type hexanoic acid may be included in 20 parts by weight or more, 30 parts by weight or more, 40 parts by weight or more, 50 parts by weight or more, and 95 parts by weight or less, 90 parts by weight or less, 85 parts by weight or less, 80 parts by weight or less, or 70 parts by weight or less of with respect to total 100 parts by weight of the mixture.

In addition, 1-hexanoic acid may be included in 80 parts by weight or less, 70 parts by weight or less, 60 parts by weight or less, 50 parts by weight or less, 40 parts by weight or less, or 30 parts by weight or less, and 1 part by weight or more, 2 parts by weight or more, 5 parts by weight or more or 10 parts by weight or more with respect to total 100 parts by weight of the hexanoic acid isomer mixture.

The amounts included of the branch-type and linear-type may be suitably controlled according to the application use of the triester-based plasticizer, and through the control of the ratios, desired physical properties could be achieved.

Further, the isomer mixture may further include cyclopentyl methanoic acid, and in this case, may be included in 30 parts by weight or less with respect to total 100 parts by weight of the isomer mixture. Preferably, 20 parts by weight or less, 15 parts by weight or less may be included. In the case of the cyclopentyl methanoic acid, the improvement of processability and the improvement of mechanical properties could be achieved only if included, and the amount thereof may be controlled considering the deterioration of physical properties according to the reduction of the relative contents of other isomers.

In the hexanoic acid isomer mixture determining the degree of branching of the plasticizer composition according to an embodiment of the present invention, various isomers may be included, typically four types of isomers as referred to, without excluding the presence of other isomers. For example, 4-methylpentanoic acid, 2-ethylbutanoic acid, or 2, 3-dimethylbutanoic acid, may be included, and besides, the structural isomers of C6 alkyl carboxylic acid may be present.

In addition, the plasticizer composition according to an embodiment of the present invention is derived from the reaction of the above-described hexanoic acid isomer mixture and a trihydric alcohol, and the trihydric alcohol may be a glycerol-based compound and may be represented by, for example, Formula 2 below.

[Formula 2]

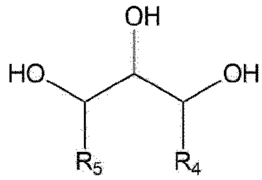

In Formula 2, $R_4$ and $R_5$ are the same as defined in Formula 1.

$R_4$ and $R_5$ may be each independently hydrogen or an alkyl group of 1 to 4 carbon atoms, preferably, hydrogen, a methyl group or an ethyl group, more preferably, hydrogen or a methyl group, and most preferably, glycerol where both $R_4$ and $R_5$ are hydrogen. The glycerol may contribute to improving the price competitiveness of the plasticizer considering that it is easy to supply, it could be synthesized from a natural material, and it is a material easily obtained from other synthetic methods.

A method of preparing the plasticizer composition according to an embodiment of the present invention is a method well-known in the art, and any methods that may prepare the aforementioned plasticizer composition may be applied, without specific limitation.

That is, by suitably controlling esterification reaction, the plasticizer composition according to the present invention may be prepared. For example, the composition may be prepared by the direct esterification of a hexanoic acid isomer mixture with the glycerol-based compound represented by Formula 2, for example, glycerol.

The plasticizer composition according to an embodiment of the present invention is a material prepared by suitably performing the esterification reaction, and any preparation methods satisfying the aforementioned conditions, specifically, controlling the ratio of the branch-type hexanoic acid in the isomer mixture, may be applied, without specific limitation.

For example, the direct esterification may be performed by: a step of injecting a hexanoic acid isomer mixture and a glycerol-based compound represented by Formula 2, adding a catalyst and reacting under a nitrogen atmosphere; a step of removing unreacted alcohol and neutralizing unreacted acid; and a step of dehydrating by distillation under a reduced pressure and filtering.

The case of the hexanoic acid isomer mixture, i.e., monocarboxylic acid, may perform the main function determining the component ratio in the composition prepared, and the theoretical molar ratio of 3:1 with the glycerol-based compound may be applied. If the hexanoic acid isomer mixture greater than the molar ratio is additionally injected, the reaction rate may be improved. In this case, the additional injection amount of the hexanoic acid isomer mixture may be 400 mol% or less, or 300 mol% or less, preferably, 200 mol% or less or 100 mol% or less with respect to the equivalent of the hexanoic acid isomer mixture.

The catalyst may be, for example, at least one or more selected from an acid catalyst such as sulfuric acid, hydrochloric acid, phosphoric acid, nitric acid, paratoluenesulfonic acid, methanesulfonic acid, ethanesulfonic acid, propanesulfonic acid, butanesulfonic acid, and alkyl sulfate, a metal salt such as aluminum lactate, lithium fluoride, potassium chloride, cesium chloride, calcium chloride, iron chloride, and aluminum phosphate, a metal oxide such as a heteropoly acid, natural/synthetic zeolites, cation and anion exchange resins, and an organometal such as tetraalkyl titanate and polymers thereof. In a particular embodiment, the catalyst may use tetraalkyl titanate. Preferably, as an acid catalyst having a low activation temperature, paratoluenesulfonic acid and methanesulfonic acid may be suitable.

The amount used of the catalyst may be different according to the types, and for example, a homogeneous catalyst may be used in a range of 0.01 to 5.00 wt%, 0.01 to 3.00 wt%, 0.1 to 3.0 wt% or 0.1 to 2.0 wt% based on total 100 wt% of reactants, and a heterogeneous catalyst may be used in a range of 5 to 200 wt%, 5 to 100 wt%, 20 to 200 wt%, or 20 to 150 wt% based on the total amount of the reactants.

In this case, the reaction temperature may be within a range of 100 to 280°C, 100 to 250°C, or 100 to 230°C.

According to another embodiment of the present invention, a resin composition including the plasticizer composition and a resin is provided.

The resin may use resins well-known in the art. For example, a mixture of one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, natural rubber, synthetic rubber and thermoplastic elastomer may be used, without limitation.

The plasticizer composition may be included in 5 to 150 parts by weight, preferably, 5 to 130 parts by weight, or 10 to 120 parts by weight based on 100 parts by weight of the resin.

Generally, the resin using the plasticizer composition may be prepared into a resin product through a melt processing or a plastisol processing, and a resin by the melt processing and a resin from the plastisol processing may be produced differently according to each polymerization method.

For example, in case of using a vinyl chloride polymer in a melt processing, solid phase resin particles having a large average particle diameter are prepared by suspension polymerization, are used, and the vinyl chloride polymer is referred to as a straight vinyl chloride polymer. In case of using a vinyl chloride polymer in a plastisol processing, a sol state resin as minute resin particles are prepared by emulsion polymerization, are used, and this vinyl chloride polymer is referred to as a paste vinyl chloride resin.

In case of the straight vinyl chloride polymer, a plasticizer may be included in a range of 5 to 80 parts by weight with respect to 100 parts by weight of the polymer, and in case of the paste vinyl chloride polymer, the plasticizer may be included in a range of 40 to 120 parts by weight with respect to 100 parts by weight of the polymer.

The resin composition may further include a filler. The filler may be 0 to 300 parts by weight, preferably, 50 to 200 parts by weight, more preferably, 100 to 200 parts by weight based on 100 parts by weight of the resin.

The filler may use fillers well-known in the art and is not specifically limited. For example, the filler may be a mixture of one or more kinds selected from silica, magnesium carbonate, calcium carbonate, hard coal, talc, magnesium hydroxide, titanium dioxide, magnesium oxide, calcium hydroxide, aluminum hydroxide, aluminum silicate, magnesium silicate and barium sulfate.

In addition, the resin composition may further include other additives such as a stabilizer as necessary. Each of the other additives such as the stabilizer may be, for example, 0 to 20 parts by weight, preferably, 1 to 15 parts by weight based on 100 parts by weight of the resin.

The stabilizer may use, for example, a calcium-zinc-based (Ca-Zn-based) stabilizer such as a composite stearate of calcium-zinc or a barium-zinc-based (Ba-Zn-based) stabilizer, but is not specifically limited.

The resin composition may be applied to both a melt processing and a plastisol processing as described above, and a calendaring processing, an extrusion processing, or an injection processing may be applied to the melt processing, and a coating processing,

## Examples

Hereinafter, embodiments will be explained in detail to particularly explain the present invention. The present invention may, however, be embodied in different forms and should not be construed as limited to the embodiments set forth herein. Rather, these embodiments are provided so that this disclosure will be thorough and complete, and will fully convey the scope of the inventive concept to those skilled in the art.

### Example 1

To a reactor equipped with a stirrer, a condenser and a decanter, 1360 g of a mixture including about 9 wt% of 1-hexanoic acid, about 35 wt% of 2-methylpentanoic acid, about 44 wt% of 3-methylpentanoic acid, about 7 wt% of 4-methylpentanoic acid and about 5 wt% of cyclopentyl methanoic acid, as a hexanoic acid isomer mixture, 276 g of glycerol and 5 g of methanesulfonic acid were added, and esterification reaction was performed at a reaction temperature of 100 to 140°C under a nitrogen atmosphere. After finishing the reaction, an unreacted acid was removed, a catalyst and a product were neutralized with an alkaline aqueous solution, and washed. An unreacted raw material and moisture were separated to finally obtain a triester-based plasticizer composition.

### Examples 2

A triester-based plasticizer composition was obtained by the same method as in Example 1 except for using 1360 g of a mixture including about 20 wt% of 1-hexanoic acid, about 30 wt% of 2-methylpentanoic acid, about 35 wt% of 3-methylpentanoic acid, about 5 wt% of 4-methylpentanoic acid and about 10 wt% of cyclopentyl methanoic acid, as the hexanoic acid isomer mixture.

### Examples 3

A triester-based plasticizer composition was obtained by the same method as in Example 1 except for using 1360 g of a mixture including about 2 wt% of 1-hexanoic acid, about 40 wt% of 2-methylpentanoic acid, about 50 wt% of 3-methylpentanoic acid, about 2 wt% of 4-methylpetanoic acid and about 6 wt% of cyclopentyl methanoic acid, as the hexanoic acid isomer mixture.

### Examples 4

A triester-based plasticizer composition was obtained by the same method as in Example 1 except for using 1360 g of a mixture including about 5 wt% of 1-hexanoic acid, about 50 wt% of 2-methylpentanoic acid, about 30 wt% of 3-methylpentanoic acid, and about 15 wt% of cyclopentyl methanoic acid, as the hexanoic acid isomer mixture.

**Comparative Example 1**

Dioctyl phthalate (DOP, LG Chem,) was used as a plasticizer.

**Comparative Example 2**

Diisononyl phthalate (DINP), LG Chem,) was used as a plasticizer.

**Comparative Example 3**

GL300 which is a product of LG Chem, and is a dioctyl terephthalate, was used as a plasticizer.

**Comparative Example 4**

GL500 which is a product of LG Chem, and is a mixture of dibutyl terephthalate, butyloctyl terephthalate and dioctyl terephthalate, was used as a plasticizer.

**Comparative Example 5**

A triester-based plasticizer composition was obtained by the same method as in Example 1 except for using 1360 g of an acid mixture of n-butanoic acid and benzoic acid in a weight ratio of 7:3 instead of the hexanoic acid isomer mixture.

**Comparative Example 6**

A triester-based plasticizer composition was obtained by the same method as in Example 1 except for using 1360 g of a single compound of 3-methylpentanoic acid instead of the hexanoic acid isomer mixture.

**Comparative Example 7**

A triester-based plasticizer composition was obtained by the same method as in Example 1 except for using 1030 g of n-butanoic acid instead of the hexanoic acid isomer mixture.

**Comparative Example 8**

A triester-based plasticizer composition was obtained by the same method as in Example 1 except for using 1523 g of n-heptanoic acid instead of the hexanoic acid isomer mixture.

**Comparative Example 9**

A triester-based plasticizer composition was obtained by the same method as in Example 1 except for using 1520 g of an acid mixture of 1-hexanoic acid and 2-ethylhexanoic acid in 50 wt% each instead of the hexanoic acid isomer mixture.

The type and amount used of the acid used in the Examples and Comparative Examples, and the degree of branching of the acid mixtures are summarized in Table 1 below.

[Table 1]

| | Type and amount of acid (%) | | | | | Degree of branching |
|---|---|---|---|---|---|---|
| | 1-Hexanoic acid | 2-Methylpentanoic acid | 3-Methylpentanoic acid | 4-Methylpentanoic acid | Cyclopentyl methanoic acid | |
| Example 1 | 9 | 35 | 44 | 7 | 5 | 0.86 |
| Example 2 | 20 | 30 | 35 | 5 | 10 | 0.7 |
| Example 3 | 2 | 40 | 50 | 2 | 6 | 0.92 |

(continued)

| | Type and amount of acid (%) | | | | | Degree of branching |
|---|---|---|---|---|---|---|
| | 1-Hexanoic acid | 2-Methylpentanoic acid | 3-Methylpentanoic acid | 4-Methylpentanoic acid | Cyclopentyl methanoic acid | |
| Example 4 | 5 | 50 | 30 | 0 | 15 | 0.8 |
| Comparative Example 1 | - | | | | | - |
| Comparative Example 2 | - | | | | | - |
| Comparative Example 3 | - | | | | | - |
| Comparative Example 4 | - | | | | | - |
| Comparative Example 5 | 70 wt% of n-butanoic acid and 30 wt% of benzoic acid | | | | | 0 |
| Comparative Example 6 | 3-methylpentanoic acid alone | | | | | 1 |
| Comparative Example 7 | n-butanoic acid alone | | | | | 0 |
| Comparative Example 8 | n-heptanoic acid alone | | | | | 0 |
| Comparative Example 9 | 50 wt% of 1-hexanoic acid and 50 wt% of 2-ethylhexanoic acid | | | | | 1 |

**Experimental Example 1: Evaluation of sheet performance**

By using the plasticizers of the Examples and Comparative Examples, specimens were manufactured according to ASTM D638 and the prescription and manufacturing conditions below.

**(1) Prescription:** 100 parts by weight of a straight vinyl chloride polymer (LS100), 50 parts by weight of a plasticizer and 3 parts by weight of a stabilizer (BZ-153T)
**(2) Mixing:** mixing at 98°C in 700 rpm
**(3) Manufacture of specimen:** 1T, 2T and 3T sheets were manufactured by processing at 160°C for 4 minutes by a roll mill, and at 180°C for 2.5 minutes (low pressure) and 2 minutes (high pressure) by a press.
**(4) Test items**

1) Hardness: Shore hardness (Shore "A" and "D") at 25°C was measured using a 3T specimen for 10 seconds using ASTM D2240. The plasticization efficiency was assessed excellent if the value was small.
2) Tensile strength: By an ASTM D638 method, a specimen was drawn in a cross-head speed of 200 mm/min using a test apparatus of U.T.M (manufacturer: Instron, model name: 4466), and a point where the 1T specimen was cut was measured. The tensile strength was calculated by Mathematical Formula 1 below.

$$\text{Tensile strength (kgf/cm}^2\text{)} = \text{load value (kgf)/thickness (cm)} \times \text{width (cm)} \qquad \text{[Mathematical Formula 1]}$$

3) Elongation rate measurement: By an ASTM D638 method, a specimen was drawn in a cross-head speed of 200 mm/min using a test apparatus of U.T.M, and a point where the 1T specimen was cut was measured. The elongation rate was calculated by Mathematical Formula 2 below.

$$\text{Elongation rate (\%)} = \text{length after elongation/initial length} \times 100 \qquad \text{[Mathematical Formula 2]}$$

4) Migration loss measurement: According to KSM-3156, a specimen with a thickness of 2 mm or more was obtained, glass plates were attached onto both sides of 1T specimen, and a load of 1 kgf/$cm^2$ was applied. The specimen was stood in a hot air circulation type oven (80°C) for 72 hours and then taken out and cooled at room temperature for 4 hours. Then, the weights of the specimen from which glass plates attached onto both sides thereof were removed, were measured before and after standing the glass plates and the specimen plate in the oven, and the migration loss was calculated by Mathematical Formula 3 below.

Migration loss (%) = [{ (weight of initial specimen) - (weight of specimen after standing in oven) }/ (weight of initial specimen)] × 100 [Mathematical Formula 3]

5) Volatile loss measurement: The specimen manufactured was processed at 80°C for 72 hours, the weight of the specimen was measured, and measurement was performed by Mathematical Formula 4 below.

Volatile loss (wt%) = [{ (weight of initial specimen) - (weight of specimen after processing) }/(weight of initial specimen)] × 100 [Mathematical Formula 4]

6) Stress test (stress resistance): A specimen with a thickness of 2 mm in a bent state was stood at 23°C for 168 hours, and the degree of migration (degree of oozing) was observed. The results were recorded as numerical values, and excellent properties were shown if the value was closer to 0.

7) Absorption rate measurement

Absorption rate was evaluated by measuring the time consumed for mixing a resin and an ester compound and stabilizing the torque of a mixer by using a planetary mixer (Brabender, P600) in conditions of 73°C and 60 rpm. For reference, if the absorption rate is measured as less than 4 minutes, it seems that the absorption and migration of a plasticizer are repeatedly carried out during processing, and if the absorption rate is greater than 9 minutes, it is considered that the absorption itself is hardly carried out. Accordingly, if a value between 4 minutes to 9 minutes is not measured, it is evaluated as impossible to process.

**(5) Evaluation results**

The evaluation results on the test items are shown in Table 2 below.

[Table 2]

| | Hardness | | Tensile strength (kgf/$cm^2$ ) | Elongation rate (%) | Migration loss (%) | Volatile loss (%) | Stress resis tance | Absorption rate (mm: ss) |
|---|---|---|---|---|---|---|---|---|
| | (Shore A) | (Shore D) | | | | | | |
| Example 1 | 80.6 | 34.1 | 201.8 | 336.4 | 4.54 | 2.50 | 0.5 | 4:18 |
| Example 2 | 80.4 | 34.0 | 205.7 | 335.8 | 4.20 | 2.10 | 0.5 | 4:20 |
| Example 3 | 80.7 | 34.5 | 204.9 | 332.5 | 4.62 | 2.60 | 0.5 | 4:15 |
| Example 4 | 80.5 | 34.2 | 207.8 | 339.8 | 4.00 | 2.00 | 0.5 | 4:20 |
| Comparative Example 1 | 83.9 | 38.4 | 195.1 | 323.4 | 1.53 | 1.57 | 0.5 | 5:30 |
| Comparative Example 2 | 85.9 | 40.1 | 203.7 | 323.4 | 2.47 | 0.73 | 0.5 | 6:46 |
| Comparative Example 3 | 87.7 | 41.6 | 206.0 | 335.8 | 6.26 | 0.82 | 3.0 | 7:30 |
| Comparative Example 4 | 84.2 | 38.8 | 207.7 | 330.4 | 5.36 | 3.03 | 1.5 | 5:20 |
| Comparative Example 5 | 80.8 | 36.8 | 202.5 | 318.6 | 0.23 | 6.01 | 0.5 | Impossible to process |

(continued)

| | Hardness | | Tensile strength (kgf/cm$^2$ ) | Elongation rate (%) | Migration loss (%) | Volatile loss (%) | Stress resis tance | Absorption rate (mm: ss) |
|---|---|---|---|---|---|---|---|---|
| | (Shore A) | (Shore D) | | | | | | |
| Comparative Example 6 | 80.7 | 34.5 | 200.1 | 321.0 | 4.87 | 3.21 | 0.5 | 4:15 |
| Comparative Example 7 | 76.1 | 32.5 | 187.6 | 284.3 | 5.88 | 12.40 | 0.5 | Impossible to process |
| Comparative Example 8 | 83.8 | 36.4 | 210.3 | 310.7 | 8.47 | 1.54 | 1.5 | 4:56 |
| Comparative Example 9 | 84.0 | 37.0 | 211.5 | 320.4 | 7.86 | 1.88 | 2 | 5:25 |

Referring to the results of Table 2, it could be confirmed that the plasticizer compositions according to embodiments of the present invention showed very excellent plasticization efficiency and noticeably improved absorption rate even with markedly high elongation rate when compared to Comparative Examples 1 and 2, which are the conventional phthalate-based products, and big improvements were observed on plasticization efficiency, migration loss and volatile loss, further, stress resistance even compared to Comparative Examples 3 and 4, which are eco-friendly products. In addition, through the excellent plasticization efficiency and absorption rate simultaneously, it could be confirmed that the plasticizer compositions of the present invention have very excellent processability, are suitable for mass production, and are stable products.

Also, it could be confirmed that the plasticizer compositions of the present invention accomplished the equal or better levels when compared to the plasticizers of Comparative Examples 1 and 2, which are the conventional phthalate-based plasticizers with high performance but inducing fatal environmental issues, and are very suitable as substitutes.

In addition, Comparative Example 5 in which an esterification product of glycerol and an acid was used, but a mixture of n-butanoic acid and benzoic acid other than the hexanoic acid isomer mixture was used as an acid, showed markedly low elongation rate in contrast to the Examples of the present invention, and showed markedly inferior results in view of volatile loss in contrast to the Examples of the present invention. Further, in the case of Comparative Example 5, in the experiment for measuring absorption rate, impossible results to process were shown. In addition, Comparative Example 9 in which a mixture of hexanoic acid and 2-ethylhexanoic acid was applied as an acid, showed markedly inferior plasticization efficiency in contrast to the Examples, and inferior elongation rate, migration loss, stress resistance and absorption rate in contrast to the Examples. From the results, it could be confirmed that the improving effects accomplished through the plasticizer composition of the present invention were not due to the application of other acids but due to the application of the hexanoic acid of 6 carbon number, particularly, confirmed due to the use of the hexanoic acid in an isomer mixture type.

Meanwhile, Comparative Example 6 in which a hexanoic acid was used, but this was used not as an isomer mixture type but as a single compound, showed inferior elongation rate in contrast to the Examples, and showed slightly inferior volatile loss in contrast to the Examples. In addition, Comparative Example 7 in which n-butanoic acid with 4 carbon number was used, showed markedly inferior results of all aspects of tensile strength, elongation rate, migration loss and volatile loss in contrast to the Examples, and also showed impossible to process for absorption rate. Finally, Comparative Example 8 in which n-heptanoic acid with 7 carbon number was used, showed markedly inferior plasticization efficiency due to high hardness in contrast to the Examples, and showed inferior results on elongation rate, migration loss and stress resistance in contrast to the Examples. The absorption rate was also slightly inferior in contrast to the Examples. From the results, it was confirmed that the hexanoic acid isomer mixture was required to use as in the Examples of the present invention to achieve excellent plasticization efficiency, mechanical properties, and various properties such as stress resistance and processability in balance, and such improving effects could not be achieved in the case of applying acids with a carbon number other than hexanoic acid (Comparative Examples 7 and 8), or in the case of applying only one type of hexanoic acid (Comparative Example 6).

**Experimental Example 2: Evaluation of plastisol performance**

By using the plasticizers of the Examples and Comparative Examples, specimens were manufactured according to ASTM D638 and the prescription and manufacturing conditions below.

**(1) Prescription:** 100 parts by weight of a paste vinyl chloride polymer (KH-10), 70 parts by weight of a plasticizer, 3

parts by weight of a stabilizer (BZ-119), 3 parts by weight of a foaming agent (AC5000) and 40 parts by weight of a filler (OMYA-10)

**(2) Mixing:** mixing at 1000 rpm for 15 minutes

**(3) Test items**

1) Viscosity: Measurement was performed as Brookfield viscosity, using a Brookfield (LV type) viscometer, #64 was used as a spindle, measurement rate was 6 rpm and 60 rpm, and measurement temperatures were 25°C and 40°C.

**(4) Evaluation results**

The evaluation results on the test items are shown in Table 3 below.

[Table 3]

| | 25°C/6 rpm | | | 25°C/60 rpm | | | 40°C/6 rpm | | | 40°C/60 rpm | | |
|---|---|---|---|---|---|---|---|---|---|---|---|---|
| | 1 hr | 24 hr | Δ | 1 hr | 24 hr | Δ | 1 hr | 24 hr | Δ | 1 hr | 24 hr | Δ |
| Example 1 | 500 | 800 | 300 | 470 | 570 | 100 | 300 | 120 0 | 900 | 370 | 740 | 370 |
| Example 2 | 500 | 800 | 300 | 450 | 550 | 100 | 300 | 1100 | 800 | 300 | 650 | 350 |
| Example 3 | 450 | 700 | 250 | 450 | 500 | 50 | 350 | 1050 | 700 | 300 | 600 | 300 |
| Example 4 | 450 | 650 | 200 | 450 | 520 | 70 | 330 | 1030 | 700 | 300 | 650 | 350 |
| Comparative Example 1 | 1700 | 2700 | 1000 | 1930 | 2340 | 410 | 1500 | 2500 | 1000 | 1180 | 2060 | 880 |
| Comparative Example 2 | 2300 | 2400 | 100 | 2080 | 2570 | 490 | 1700 | 2700 | 1000 | 1270 | 1710 | 440 |
| Comparative Example 3 | 2200 | 2300 | 100 | 2020 | 2300 | 280 | 1300 | 1900 | 600 | 1220 | 1450 | 230 |
| Comparative Example 4 | 1200 | 1900 | 700 | 1380 | 1650 | 270 | 1200 | 1900 | 700 | 870 | 1390 | 520 |
| Comparative Example 5 | 1900 | 2400 | 500 | 1920 | 2440 | 520 | 1600 | 10600 | 9000 | 1520 | 7390 | 5870 |
| Comparative Example 6 | 500 | 800 | 300 | 500 | 600 | 100 | 400 | 1400 | 1000 | 350 | 750 | 400 |
| Comparative Example 7 | 2500 | 3000 | 500 | 2100 | 3200 | 1100 | 1800 | 12800 | 11000 | 2400 | 9000 | 6600 |
| Comparative Example 8 | 450 | 700 | 250 | 400 | 600 | 200 | 300 | 900 | 600 | 300 | 700 | 400 |
| Comparative Example 9 | 600 | 1000 | 400 | 600 | 700 | 100 | 350 | 1400 | 1050 | 350 | 750 | 400 |

Referring to the results of Table 3, it could be found that the plasticizer compositions of Examples 1 to 4 showed very low initial viscosity themselves during plastisol processing, and processing was significantly favorable, and showed a small viscosity change according to time, and viscosity stability was excellent. However, it could be found that Comparative Examples 1 to 4, which correspond to the conventional products, showed high viscosity themselves, and plastisol processing was very disadvantageous in contrast to the Examples. Particularly, in the cases of Comparative Examples 1 to 4, the viscosity change was large as well as an initial viscosity, and it could be confirmed that performance was markdely inferior in the plastisol processing in contrast to the plasticizer composition of the present invention.

Meanwhile, it could be confirmed that Comparative Examples 5 to 9, in which the esterification reaction of glycerol was used for preparing plasticizer compositions similar to the plasticizer composition of the present invention, but different acids were used, showed inferior performance in a plastisol processing in contrast to the Examples of the present

invention. Particularly, in the case of Comparative Example 5 in which n-butanoic acid and benzoic acid were mixed and used, an initial viscosity was higher by about 4 times in contrast to the Examples of the present invention, and processing itself was impossible, and the viscosity change according to time was also high, and viscosity stability was also deteriorated. In the case of Comparative Example 6 in which an acid with 6 carbon number was used similar to the Examples of the present invention, but 3-methylpentanoic acid was used alone instead of an isomer mixture, both an initial viscosity and viscosity stability were shown similar levels as the Examples, but under plastisol processing conditions at relatively high temperature conditions of 40°C, it could be confirmed that the viscosity change according to time was increased slightly in contrast to the plasticizer compositions of the Examples. From the results, it could be inferred that if an acid with 6 carbon number is applied, but in an isomer mixture type, improving effects of viscosity stability could be achieved. In addition, in the case of Comparative Example 6 in which an acid with 4 carbon number was used, the highest initial viscosity was shown, and it was found that Comparative Example 6 was unsuitable for a plastisol processing. In the case of Comparative Example 8 in which an acid with 7 carbon number was used, an initial viscosity and viscosity stability at low rpm were similar to those of the Examples, but viscosity stability at high rpm was inferior to the that of the Examples. Finally, Comparative Example 9 in which hexanoic acid with 6 carbon number was used but used by mixing with 2-ethylhexanoic acid with 8 carbon number, showed slightly inferior results on an initial viscosity and viscosity stability overall in contrast to the Examples.

From the results, it could be confirmed that the plasticizer composition of the present invention uses an acid with 6 carbon number as an isomer mixture type and could achieve excellent processability and viscosity stability even in a plastisol processing.

## Claims

**1.** A triester-based plasticizer composition, comprising:

one or more triesters of the following Formula 1,
wherein $R_1$ to $R_3$ of Formula 1 are derived from a hexanoic acid isomer mixture having a degree of branching of 2.0 or less:

[Formula 1]

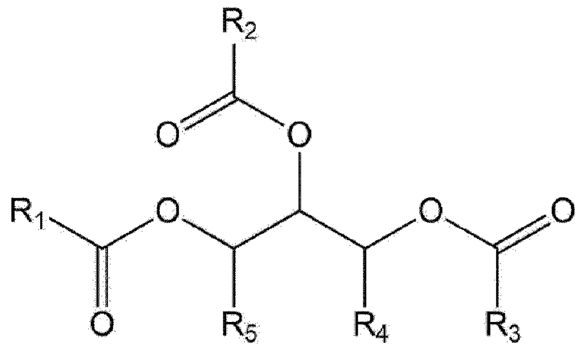

in Formula 1,
$R_1$ to $R_3$ are each independently an n-pentyl group, a branch-type pentyl group or a cyclopentyl group, and
$R_4$ and $R_5$ are each independently hydrogen or an alkyl group of 1 to 4 carbon atoms.

**2.** The plasticizer composition according to claim 1, wherein the hexanoic acid isomer mixture has the degree of branching of 1.5 or less.

**3.** The plasticizer composition according to claim 1, wherein the hexanoic acid isomer mixture comprises 2-methylpentanoic acid and 3-methylpentanoic acid.

**4.** The plasticizer composition according to claim 1, wherein the hexanoic acid isomer mixture comprises 1-hexanoic acid, 2-methylpentanoic acid, 3-methylpentanoic acid and cyclopentyl methanoic acid.

**5.** The plasticizer composition according to claim 1, wherein the hexanoic acid isomer mixture comprises 20 to 95 parts by weight of a branch-type hexanoic acid with respect to total 100 parts by weight of the mixture.

6. The plasticizer composition according to claim 1, wherein the hexanoic acid isomer mixture comprises 30 parts by weight or less of cyclopentyl methanoic acid with respect to total 100 parts by weight of the mixture.

7. The plasticizer composition according to claim 1, wherein the hexanoic acid isomer mixture comprises 80 parts by weight or less of 1-hexanic acid with respect to total 100 parts by weight of the mixture.

8. The plasticizer composition according to claim 1, wherein $R_4$ and $R_5$ are hydrogen.

9. A resin composition comprising:
100 parts by weight of a resin; and 5 to 150 parts by weight of the plasticizer composition according to claim 1.

10. The resin composition according to claim 9, wherein the resin is one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, natural rubber, and synthetic rubber.

**Patentansprüche**

1. Weichmacherzusammensetzung auf Triesterbasis, umfassend:

einen oder mehrere Triester der folgenden Formel 1,
wobei $R_1$ bis $R_3$ der Formel 1 von einer Hexansäure-Isomermischung abgeleitet sind, welche einen Verzweigungsgrad von 2,0 oder weniger aufweist:

[Formel 1]

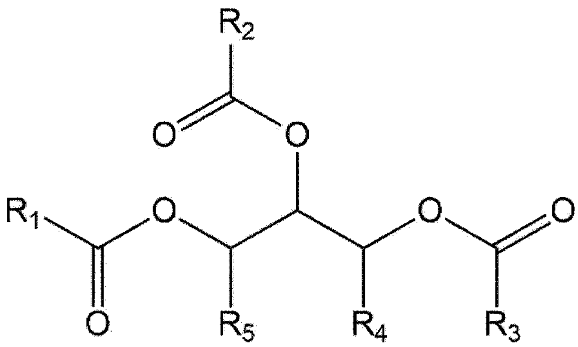

,

wobei in Formel 1
$R_1$ bis $R_3$ jeweils unabhängig eine n-Pentylgruppe, eine verzweigte Pentylgruppe oder eine Cyclopentylgruppe sind, und
$R_4$ und $R_5$ jeweils unabhängig Wasserstoff oder eine Alkylgruppe mit 1 bis 4 Kohlenstoffatomen sind.

2. Weichmacherzusammensetzung nach Anspruch 1, wobei die Hexansäure-Isomermischung den Verzweigungsgrad von 1,5 oder weniger aufweist.

3. Weichmacherzusammensetzung nach Anspruch 1, wobei die Hexansäure-Isomermischung 2-Methylpentansäure und 3-Methylpentansäure umfasst.

4. Weichmacherzusammensetzung nach Anspruch 1, wobei die Hexansäure-Isomermischung 1-Hexansäure, 2-Methylpentansäure, 3-Methylpentansäure und Cyclopentylmethansäure umfasst.

5. Weichmacherzusammensetzung nach Anspruch 1, wobei die Hexansäure-Isomermischung 20 bis 95 Gewichtsteile einer verzweigten Hexansäure umfasst, bezogen auf eine Gesamtheit von 100 Gewichtsteilen der Mischung.

6. Weichmacherzusammensetzung nach Anspruch 1, wobei die Hexansäure-Isomermischung 30 Gewichtsteile oder weniger an Cyclopentylmethansäure, bezogen auf eine Gesamtheit von 100 Gewichtsteilen der Mischung, umfasst.

7. Weichmacherzusammensetzung nach Anspruch 1, wobei die Hexansäure-Isomermischung 80 Gewichtsteile oder weniger an 1-Hexansäure, bezogen auf eine Gesamtheit von 100 Gewichtsteilen der Mischung, umfasst.

8. Weichmacherzusammensetzung nach Anspruch 1, wobei $R_4$ und $R_5$ Wasserstoff sind.

9. Harzzusammensetzung, umfassend:
100 Gewichtsteile eines Harzes; und 5 bis 150 Gewichtsteile der Weichmacherzusammensetzung nach Anspruch 1.

10. Harzzusammensetzung nach Anspruch 9, wobei das Harz eines oder mehrere ist, ausgewählt aus der Gruppe bestehend aus einem linearen Vinylchloridpolymer, einem Pastenvinylchloridpolymer, einem Ethylen-Vinylacetat-Copolymer, einem Ethylenpolymer, einem Propylenpolymer, Polyketon, Polystyrol, Polyurethan, Naturkautschuk und synthetischem Kautschuk.

## Revendications

1. Composition de plastifiant à base de triester, comprenant :

un ou plusieurs triesters de la Formule 1 suivante,
dans laquelle $R_1$ à $R_3$ de Formule 1 sont issus d'un mélange isomère d'acide hexanoïque présentant un degré de ramification inférieur ou égal à 2,0 :

Formule 1

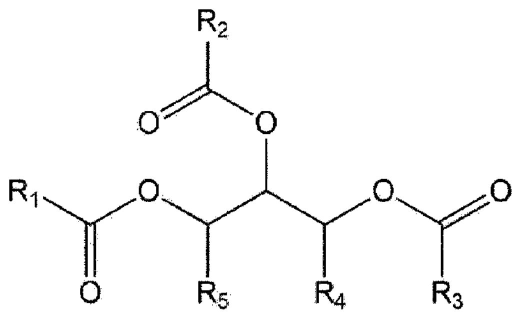

dans la Formule 1,
$R_1$ à $R_3$ sont chacun indépendamment un groupe n-pentyle, un groupe pentyle de type ramifié ou un groupe cyclopentyle, et
$R_4$ et $R_5$ sont chacun indépendamment hydrogène ou un groupe alkyle de 1 à 4 atomes de carbone.

2. Composition de plastifiant selon la revendication 1, dans laquelle le mélange isomère d'acide hexanoïque présente un degré de ramification inférieur ou égal à 1,5.

3. Composition de plastifiant selon la revendication 1, dans laquelle le mélange isomère d'acide hexanoïque comprend de l'acide 2-méthylpentanoïque et de l'acide 3-méthylpentanoïque.

4. Composition de plastifiant selon la revendication 1, dans laquelle le mélange isomère d'acide hexanoïque comprend de l'acide 1-hexanoïque, de l'acide 2-méthylpentanoïque, de l'acide 3-méthylpentanoïque et de l'acide cyclopentylméthanoïque.

5. Composition de plastifiant selon la revendication 1, dans laquelle le mélange isomère d'acide hexanoïque comprend 20 à 95 parties en poids d'un acide hexanoïque de type ramifié par rapport à un total de 100 parties en poids du mélange.

6. Composition de plastifiant selon la revendication 1, dans laquelle le mélange isomère d'acide hexanoïque comprend 30 parties en poids ou moins d'acide cyclopentylméthanoïque par rapport à un total de 100 parties en poids du mélange.

**7.** Composition de plastifiant selon la revendication 1, dans laquelle le mélange isomère d'acide hexanoïque comprend 80 parties en poids ou moins d'acide 1-hexanique, par rapport à un total de 100 parties en poids du mélange.

**8.** Composition de plastifiant selon la revendication 1, dans laquelle $R_4$ et $R_5$ sont hydrogène.

**9.** Composition de résine comprenant :
100 parties en poids d'une résine ; et de 5 à 150 parties en poids de la composition de plastifiant selon la revendication 1.

**10.** Composition de résine selon la revendication 9, dans laquelle la résine est une ou plusieurs sélectionnées dans le groupe consistant en un polymère de chlorure de vinyle droit, un polymère de chlorure de vinyle en pâte, un copolymère d'éthylène-acétate de vinyle, un polymère d'éthylène, un polymère de propylène, une polycétone, un polystyrène, un polyuréthane, un caoutchouc naturel et un caoutchouc synthétique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210080755